Europäisches Patentamt

European Patent Office

Office européen des brevets

(11) Publication number: **0 418 387 A1**

# EUROPEAN PATENT APPLICATION
## published in accordance with Art. 158(3) EPC

㉑ Application number: 89907369.6

㉒ Date of filing: 21.02.89

⑧⑥ International application number:
PCT/SU89/00046

⑧⑦ International publication number:
WO 90/09764 (07.09.90 90/21)

㉛ Int. Cl.⁵: **A61B 17/60**

㊸ Date of publication of application:
**27.03.91 Bulletin 91/13**

㉞ Designated Contracting States:
**AT BE CH DE FR GB IT LI**

㉛ Applicant: **VSESOJUZNY KURGANSKY
NAUCHNY TSENTR "VOSSTANOVITELNAYA
TRAVMATOLOGIA I ORTOPEDIA"
ul. M.Ulyanovoi, 6
Kurgan, 640005(SU)**

㉒ Inventor: **ILIZAROV, Gavriil Abramovich
ul. Klimova, 41-38
Kurgan, 640020(SU)**
Inventor: **MARKHASHOV, Abram Moiseevich
ul. Klimova, 41-28
Kurgan, 640020(SU)**

㉞ Representative: **Bibby, William Mark et al
Mathisen, Macara & co. The Coach House
6-8 Swakeleys Road
Ickenham Uxbridge UB10 8BZ(GB)**

(54) **DEVICE FOR TREATMENT OF CURVATURE OF AND DAMAGE TO THE SPINE.**

㊗ The device comprises two supporting units (1) provided with plates (2), each carrying a means for securing a spinous process (4). Said means comprises a double-toothed fork (12) with a shank (14) to be placed between two screws (3) driven into the body of the spinous process, and two securing wires (13) to be passed through the spinous process (4) and through both teeth (15) of the fork (12).

FIG.11

EP 0 418 387 A1

## DEVICE FOR TREATMENT OF SPINAL CURVATURE AND AFFECTIONS

Technical Field

The invention relates to medical engineering, more specifically to orthopedic appliances for treatment of fractures of the anterior and posterior vertebral portions, intervertebral disks and for restoration of a true shape of the vertebra, and has particular reference to devices for treatment of spinal curvatures and traumatic lesions.

Background Art

Pathology of the spine, in particular, complications secondary to spinal affections or curvatures, is one of the most sophisticated problems of traumatology and orthopedics. This is concerned with the fact that the spinal column is a polysegmental organ whose canal accommodates the spinal cord and to which major blood vessels are adjacent and vitally important interior organs are situated therealong. A majority of orthopedic devices and appliances, that is, externally applied or implantable spinal retainers are adapted to fix only the anterior or posterior vertebral portion. This fact is fraught with difficulties in elaboration of efficacious methods for treatment of some spinal diseases. It is especially difficult to retain the affected vertebrae in cases where one or more of them are completely destructed. An attemps to restore the supporting ability of the spine affected by the aforesaid traumatic lesions making use of a restorative device or transplants proves to be inadequately efficient as a result of indolent processes of bone tissue regeneration, regenerate fixation, its growing into the vertebrae, and prolonged reconstruction of the osteoplastic transplant.

A similar problem is encountered in treatment of kyphoscoliosis steaming from osteal anomaly of the ver tebral body and processes, or spina bifida occulta, i.e., due to such an osteopathy that necessitates plastic reparation of the osteal vertebral tissue.

The aforementioned difficulties encountered in solving the problem of fixing the vertebrae in cases of a traumatic injury thereto can to some extent be eliminated by the use of an implantable vertebral retainer made as a plate placed at the level of the vertebral injury and fixed by screws made fast in the vertebral bodies adjacent to the injured ones (cf.. e.g., an advertising prospectus of the firm WALDEMAR LINK GmbH, CO Wirbelsäulenplatten Modell "St.Georg" (DE).

However, implantation of the aforesaid device requires repeated major surgical intervention; though it fails to provide distraction or compression necessary for adequate reposition of the injured vertebrae.

A corset for correction of the thoracicolumbar spine (US, A, 3,889.664) is known to have gained widespread application for treatment of spine curvatures, said corset consisting of external supports in the form of girdles or belts encompassing the patient's trunk at the level of the thoracic and lumbar spines, and two distraction rods built into the supports on the other sides of the spinal curvature, which is gradually eliminated by mutual turning of the distraction rods.

However, the aforementioned construction exercises but indirect action upon the spine, which fails to attain an adequate correction.

Another type of devices for spinal curvatures are those applied to the spine under soft tissues (DE, A, 3,132,520). The device for treatment of spinal curvatures by virtue of spondylodesis for fixing two adjacent vertebrae has strips secured directly on each of the vertebrae to be fused. The strips have holes and are in terconnected by a screw thread passing in two directions and by bolts having a thread passing in the opposite directions, said bolts being separated by a central nut for a tool to apply thereto. Besides, the strips are held by bone screws that pass through the pedicle of the arch of a vertebra towards the vertebral body. Thus, by turning the connecting bolt one can set a required distance between the vertebrae being fixed.

However, such a device is implanted in soft tissues so as to affect a once-through stabilization of the vertebrae. Thus, another trauma-inflicting surgical intervention is required for correction of the intervertebral distance and removal of the device.

One more device for treatment of spinal injuries with the aid of an externally applied retainer for immobilization of the vertebrae (DE,A, 2,834,891) is known to comprise at least two supporting plates situated above the skin, each of said plates carrying at least two screws movably mounted thereon and adapted to be turned into the vertebral body, while the supporting plates themselves are interlinked through holes for adjusting their mutual position.

The device is applicable in cases of comminuted fractures of the vertebral body accompanied by damage to the intervertebral disks or their dislocation, fractures of the vertebral arches involving affection of the spinal canal, fractures of the vertebral bodies at two levels. The device for treatment of spinal injuries can operate in three modes, that is, the neutral, distracting, and compressing. Distraction and compression are attained due to elastic deformation of the Schanz's screws with the aid of rods secured in plates that adjust the mutual arrangement of said rods. In cases of vertebral fractions without affection of the height of the injured vertebra the latter is fixed by using the neutral operating mode of the device; in cases of such fractions involving affection of the vertebral height use is made of distraction operating mode of the device with application of a spongy bone graft; in cases of considerable destruction of the vertebral body and loss of the vertebral height the device is first used in the distraction mode so as to fill the defect of the vertebral body with a hard bone graft, then in the mild compression mode.

However, the aforediscussed device fails to develop the adequately high distraction and compression forces so as to form a bone regenerate necessary for restitution of the true shape of the affected vertebra and of the spinal column as a whole, which is accounted for by an insufficient connection of the vertebrae to the device, since when considerable forces are developed by the device and applied to the vertebrae involved the latter cannot practically be retained with the aid of the screws. Moreover, it is worth noting that the amount of distraction is limited to the amount of elastic deformation of the screws, which is established with the aid of the rods that interconnect the plates.

Object and Summary of the Invention

It is a primary and essential object of the present invention to provide such a device for treatment of spinal curvature and affections that would make it possible to apply considerable distraction and compression forces due to a sufficient connection of the vertebrae to the device.

Said object is accomplished due to the provision of a device for treatment of spinal curvature and affections, having at least two supporting units situated above the patient's skin, each of them comprising a plate on which at least two screws turnable into the vertebral body are movably mounted, whereas the supporting units themselves are interconnected through rods adapted for adjusting the mutual arrangement of said units, wherein, according to the invention, each of the supporting plates is provided with at least means for grasping the spinous vertebral process, said means incorporating a two-prong fork having a shank interposed between the screws on the supporting plate so as to perform a relative transverse motion for the fork prongs to straddle the spinous vertebral process, and two fixing pins passed through the spinous vertebral process and through both of the fork prongs, one of the ends of each pin being held in position on the plate, while the other end, to the fork prongs at their extension from the fork.

Provision of such a means for grasping the spinous process makes it possible, together with the screws connected to the anterior vertebral portion, to retain the posterior vertebral portion as well. The fixing pins provide for reliable grasping of the spinous process, while the fork renders it possible to transmit considerable forces to the vertebra through said pins without inflicting damage to the spinous process.

It is expedient that a slot be provided in either of the fork prongs, said slot passing between the inner and outer prong surfaces and being open towards the prong end, the longitudinal axes of the slots being inclined towards the fork longitudinal plane and being parallel to each other.

This arrangement of the slots makes it possible to fix respective ends of the pins on the prongs so as to form a space between them and the spinous vertebral process.

It is possible that each of the fork prongs have a hole that passes between the prong inner and outer surfaces, while the longitudinal axes of said holes are inclined towards the fork longitudinal plane and are parallel to each other.

Provision of such holes makes it possible, after all, to relay an effort to the vertebra to impart a backward motion thereto.

A crossarm is installed on each plate of the supporting unit parallel to said plate, said crossarm being linked to said plate through the rods adapted to adjust the distance between said plates, and the crossarms are situated on the opposite sides of the plates and are interconnected with a possibility of mutual displacement, while the rods for adjusting the mutual arrangement of the plates are connected to said plates through articulated joints.

Such a construction arrangement of the device makes possible reposition of the spinal column portions.

According to one of the embodiments of the device the crossarms are interconnected with a possibility of mutual angular motion in the frontal plane.

Such a construction arrangement of the device enables one to eliminate angular deformations of the spine in the frontal plane.

According to another embodiment of the invention one of the crossarms is shaped as a guideway, while the other crossarm is connected to the former one with a possibility of mutual parallel movement.

Such a construction arrangement renders the device applicable for treatment of spinal fractures accompanied by lateral dislocation of the vertebrae.

According to one more embodiment of the invention the guideway and the other crossarm are interconnected with a possibility of mutual angular motion in the frontal and sagittal planes.

Such a construction arrangement makes it possible to eliminate complicated angular displacements concerned with spinal deformities and fractures.

In one of the possible embodiments of the invention the device may comprise a flexible tie-member, one of its ends being rigidly coupled to the vertebra, while the other end, to the supporting units with a possibility of adjusting the tension of said flexible tie-member in a direction towards the vertex of an angle confined between the plates of the supporting units and lying in the frontal plane.

Provision of the proposed device incorporating such a tie-member provides for an additional action aimed at elimination of the lateral spinal curvature and at more uniform distribution of load imposed thereon.

According to a further embodiment of the invention the device may comprise an additional rod for adjusting the mutual arrangement of the plates, the ends of said rod being mounted on the fork prongs with a possibility of mutual displacement.

Provision of the device having an additional rod adds to rigidity of the whole structure for better elimination of lateral spinal disclocations caused by fractures of the spinal column.

According to still more embodiment of the invention the plates of the supporting units are rigidly interconnected through a bar, while at least one reposition unit is interposed between the supporting units, said reposition unit being made similar to the supporting unit and being installed on the bar with a possibility of performing an anteroposterior motion, and the plate of the reposition unit is linked to the plates of the supporting units through the rods for adjusting their mutual position.

Such a construction arrangement of the device enables one to eliminate vertebral disclocation in an anteroposterior direction and rectify spinal curvature in the sagittal plane.

According to yet still more embodiment of the invention the like ends of the plates of the supporting units are interconnected through bars at the ends of which rods are provided, adapted for adjusting the mutual position of the supporting plates, while at least one reposition unit is interposed between the supporting units, said unit being made similar to the supporting unit, and the ends of the plate of the reposition unit are connected to the respective bars with a possibility of parallel travel of the reposition unit with respect to the supporting units. Such a construction arrangement of the device is aimed at elimination of lateral disclocation of one or several vertebrae.

Brief Description of the Drawings

In what follows the device for treatment of spinal curvatures and affections is illustrated by a detailed description of some specific exemplary embodiments thereof with reference to the accompanying drawings, wherein:

FIG. 1 illustrates a supporting unit of the device, according to the invention;

FIG. 2 is a side view of an embodiment of a slotted fork;

FIG. 3 is a bottom view of FIG. 2;

FIG. 4 is a side of an embodiment of a holed fork;

FIG. 5 is a front cutaway view of FIG. 4;

FIG. 6 s a general view of the device, according to the invention;

FIG. 7 is a view of an embodiment of the device, according to the invention, wherein the supporting units can perform mutual angular motion;

FIG. 8 is a view of an embodiment of the device, according to the invention, wherein the supporting units can perform mutual parallel motion;

FIG. 9 shows an additional rod and a way of its holding to the device, according to the invention;

FIG. 10 is a view of an embodiment of the device, according to the invention, wherein the supporting units can perform angular motion in the frontal and sagittal planes;

FIG. 11 is a view of an embodiment of the device, according to the invention, incorporating a reposition unit traversable in the anteroposterior direction; and

FIG. 12 is a view of an embodiment of the device, according to the invention, wherein the reposition unit is laterally traversable.

Best Mode of Carrying Out the Invention

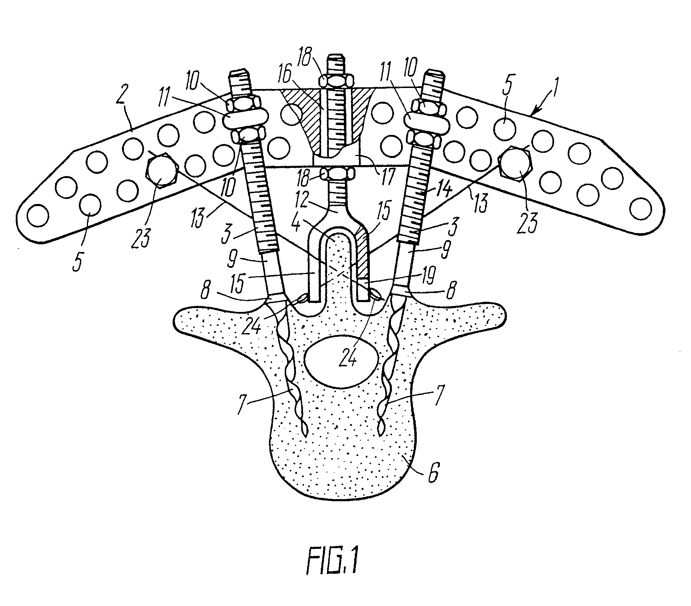

The device for treatment of spinal curvature and affections comprises at least two supporting units 1 (FIG. 1). The supporting unit 1 incorporates a plate 2, screws 3 and a means for grasping a spinous process 4. Perforations 5 are provided in the plate 2. The end of the screw 3 which interacts with a vertebral body 6 has a corkscrew thread 7 and a restrictor 8 adapted to limit the depth of penetration of the screw 3 into the vertebral body 6 for the entire period of treatment. A middle portion 9 of the screw 3 has a plain surface which is adapted to be arranged in soft tissues. The other end of the screw 3 is threaded and held with nuts 10 to a bracket 11, which is situated in the perforation 5 of the plate 2. The means for grasping the spinous process 4 incorporates a fork 12 and fixing pins 13. The fork 12 comprises a shank 14 and two prongs 15.

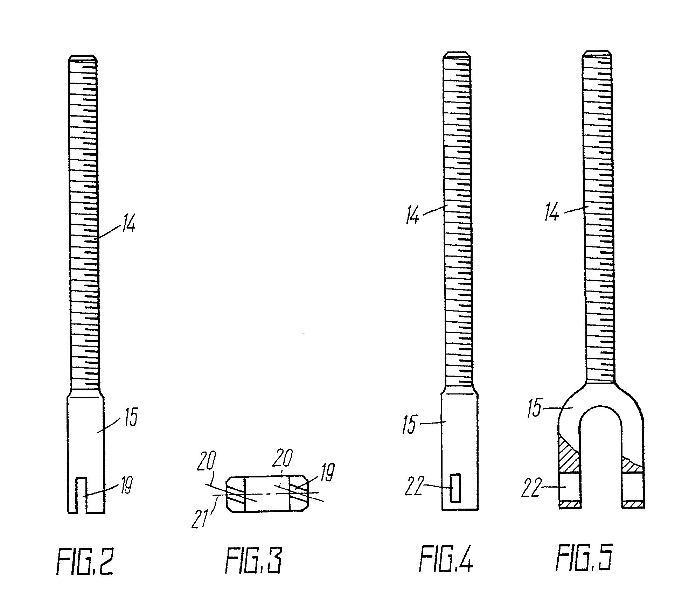

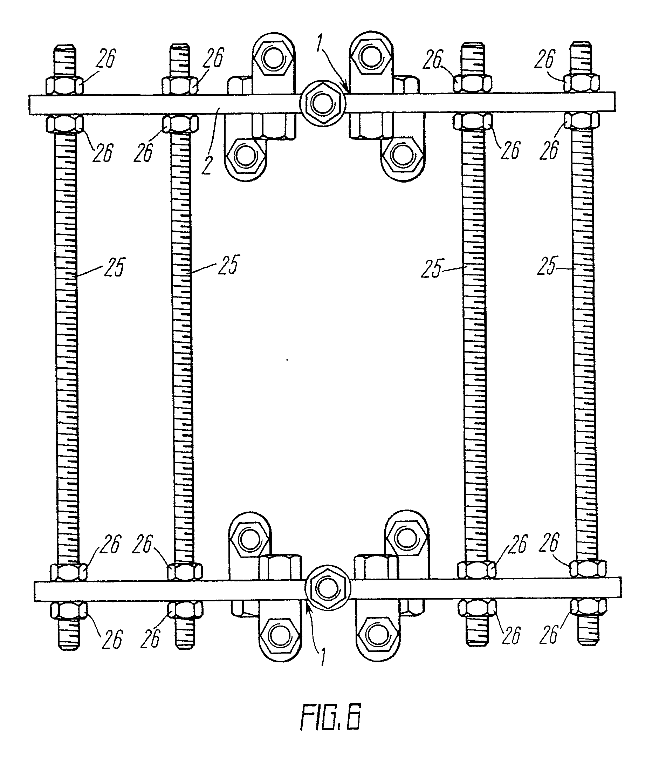

The shank 14 of the fork 12 has a thread and runs through a passage 16 made in a boss 17 of the plate 2, wherein it is held by nuts 18. The prongs 15 of the fork 12 straddle the spinous process 4. A slot 19 (FIGS 2 to 3) is made in the prong 15 of the fork 12, said slot passing between the inner and outer surfaces of the prong 15 and being open towards the end of the prong 15. Longitudinal axes 20 of the slots 19 are inclined towards a longitudinal plant 21 of the fork 12 and are parallel to each other.

According to an alternative embodiment of the fork 12, each of its prongs 15 has a hole 22, (Figs 4-5) which passes between the inner and other surfaces of the prong 15. The longitudinal axes of the holes are also inclined towards the longitudinal plane 21 of the fork 12 and are parallel to each other.

The fixing pins 13 which pass through the slots 19 or the holes 22 are spaced somewhat apart due to an inclined position of the longitudinal axes 20, the slots 19 or the holes 22 and are crossed over in the spinous process 4 at a certain gap therebetween left for the sake of less destruction of the osseous tissue.

One end of the pin 13 is held to the plate 2 with the aid of a bolt 23 which passes through one of the holes 5 provided in the plate 2, while the other end of the pin 13 runs, bypassing the nearest prong, through the slot 19 or the hole 22 in the farthest prong 15 of the fork 12 and is fixed in position by means of a stop 24 resting on the outer surface of the prong 15.

The supporting unit is fixable to one or two vertebrae depending on the therapeutic purposes to be attained.

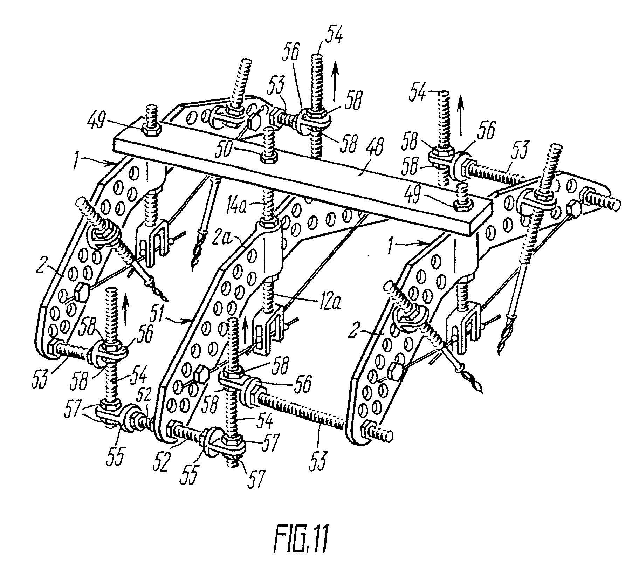

When one vertebra is to be fixed use is made of two screws 3, while if two vertebrae should be fixed, four screws 3 (FIG. 10) are employed, and the fork 12, is held to the spinous process 4 of either of the vertebrae fixed by the screws 3.

The supporting units 1 of the device, according to the invention, are interconnected through rods 25 for adjusting the mutual position of the plates 2, said rods being provided with a thread. The rods 25 pass through the holes 5 in the plate 2 and are fixed by nuts 26.

The device, according to the invention, as shown in FIGS 1 to 6 is instrumental in applying considerable distraction forces to the spine that are necessary for growing vertebral osseous tissue (that is, the bone regenerate), the body, arches, processes of a vertebra in cases of affections involving high loss of the osseous substance, in order to restore the true spinal shape. The device enables also creation of compression forces for performing an appropriate therapeutic task.

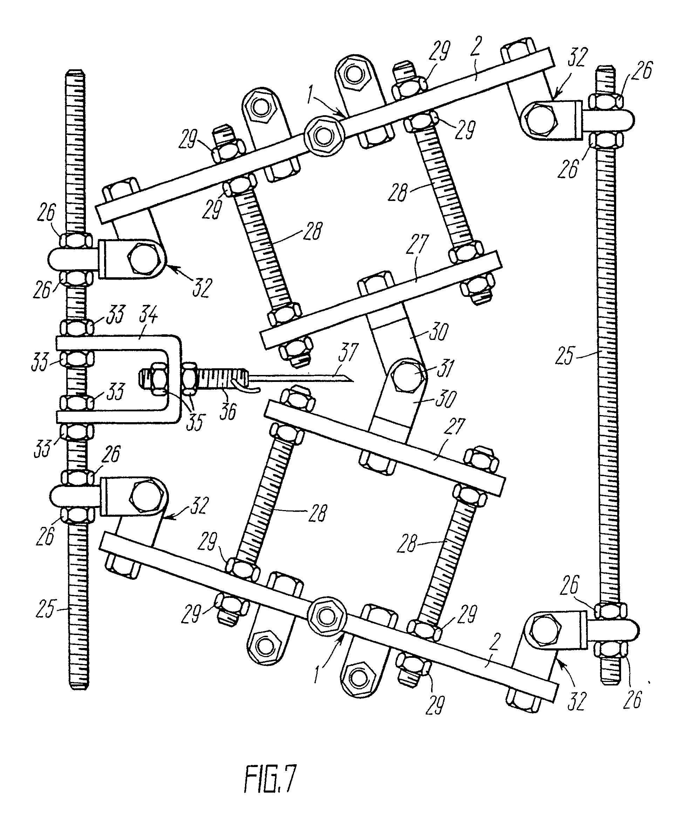

The device, according to an embodiment shown in FIG. 7, comprises two supporting units 1. A crossarm 27 made as a perforated plate is installed on each of the opposite sides of the plate 2. The crossarm 27 is connected to the plate 2 through rods 28 for adjusting the distance between the plate 2 and the crossarm 27. The rods 28 have a thread and are fixed on the crossarm 27 and the plate 2 by nuts 29. Held to the middle portion of each crossarm 27 is a bracket 30 having a hole at its free end. The bracket 30 is connected to a bolt 31 passing through the hole, thus establishing a hinge joint which provides for mutual angular motion of the supporting units 1 in the frontal plane. The rods 25 are connected to the plates 2 through hinge joints 32.

The aforesaid embodiment of the device, according to the invention, is instrumental in eliminating the lateral curvature of the spine.

According to another embodiment of the device as shown in Fig. 7, it may comprise a shackle 34 fitted on one of the rods 25 and fixed by nuts 33. A rod 36 is mounted on the shackle 34 by means of nuts 35, said rod being adapted for adjusting the tension of a flexible tie-member 37 secured on the rod 36 and made of, e.g., a metallic wire or a synthetic filament. The other end of the flexible tie-member 37 is held to the vertebra involved.

The aforementioned embodiment of the device makes it possible to exert an immediate effect on the apex of the spinal curvature in order to rectify the deformity.

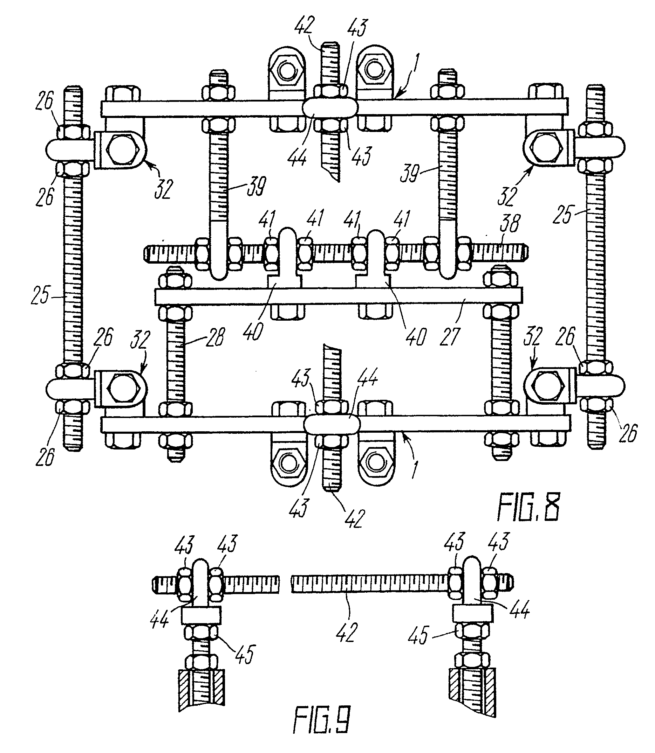

In an alternative embodiment of the device as shown in FIG. 8 one of the crossarms 27 is made as a guideway 38, which is in fact a threaded rod fitted in the lugs of rods 39 secured on the plate.

The rod of the guideway 38 passes through the holes in brackets 40 secured on the crossarms 27 of the other supporting unit 1 and is fixed therein and in the lugs of the rods 39 by means of nuts 41. In this case the supporting units 1 are capable of parallel motion with respect to each other.

The aforesaid embodiment of the invention, according to the invention, provides for reposition in cases of spinal fractures accompanied by lateral dislocation of some spinal portions.

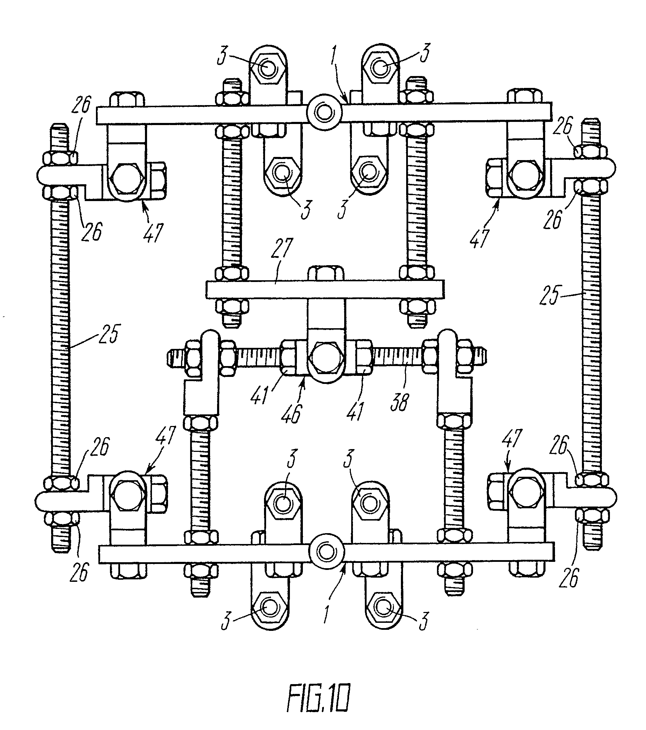

According to a further embodiment of the invention the device shown in FIG. 8 may comprise an additional rod 42 aimed at adjusting the mutual position of the plates 2. The additional rod 42 has a thread and is fixed with nuts 43 (FIG. 9) in the holes of brackets 44 which are installed at the ends of the shanks 14 of the forks 12 with a possibility of rotating round the axis of the rod 14 and are fixed by nuts 45.

Provision of the additional rod 42 makes it possible, whenever necessary, to increase rigidity of the device aimed at eliminating considerable lateral dislocations of the spinal portions.

The device, (Fig. 10) according to a preferable embodiment of the present invention, is similar to that illustrated in FIG. 8, with the sole exception that the crossarm 27 and the guideway 38, as well as the plates 2 and the rods 25 are interconnected through two respective two-axis hinge joints 46 and 47. In this case each of the supporting plates 2 carries four screws 3.

The aforesaid preferable embodiment of the device makes it possible to eliminate angular spinal curvatures in both the frontal and sagittal planes. The present embodiment of the device may be provided, whenever necessary, with the flexible tie-member 37 and a mechanism for its tensioning, as shown in FIG. 7, as well as with the additional rod 42 illustrated in FIGS 8 and 9.

FIG. 11 presents the device, according to one more embodiment of the present invention, which comprises two supporting units interlinked through a bar 48. Holes are provided at the ends of the bar 48 for accepting the shanks 14 of the forks 12, said shanks being fixed by nuts 49. A hole is provided in the middle portion of the bar 48, wherein an extended shank 14a of a fork 12a is movably mounted with the aid of nuts 50, said fork making part of a reposition unit 51, which comprises, as a rule, the same components as the supporting unit 1. The reposition unit 51 may comprise the screws 3, or may not comprise them (which is the case in FIG. 11). The plate 2a of the reposition unit 51 is connected to the plates 2 of the supporting units 1 through rods 52, 53 square with the plates 2, 2a and rods 54 parallel to said plates, said rods 52, 53, 54 being aimed at adjusting the mutual position of the plates 2, 2a. The rods 52, 53, 54 have a thread and are connected in series through brackets 55, 56 and nuts 57, 58 and are linked to the plates 2, 2a through the respective nuts 26 and 59. The device, according to the aforesaid embodiment, may comprise two or more reposition units 51, accordingly.

The aforementioned embodiment of the device, according to the invention, enables one to eliminate vertebral dislocation in the case of anterior or posterior spondylolisthesis, or dislocation of a spinal portion in the sagittal plane in cases of fractures.

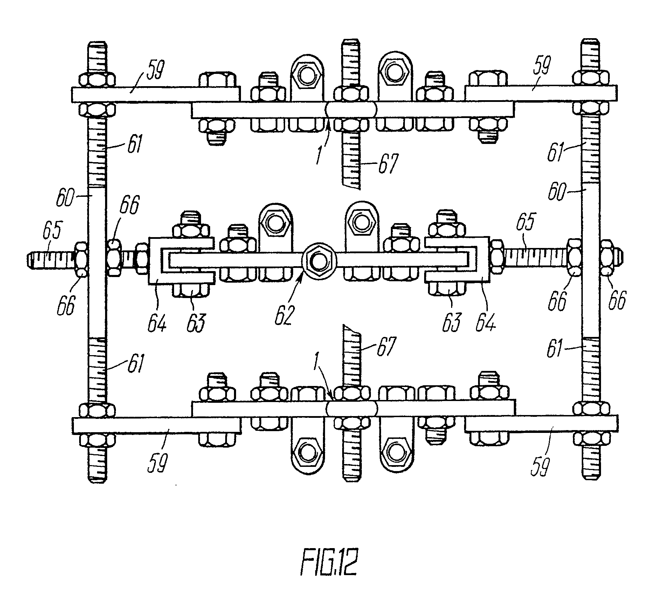

The device according to an embodiment of the invention represented in FIG. 12, comprises two supporting units 1, which incorporate strips 59 aimed at extending the plates 2. The like ends of the plates 2 are connected, via the strips 59, to bars 60 whose ends carry rods 61 adapted for adjusting the mutual position of the plates 2.

A reposition unit 62 is interposed between the supporting units 1, said reposition units being similar to the supporting unit 1 and being connected to the bars 60 through bolts 63, shackles 64, and rods 65 with nuts 66. The rods 65 pass through holes in the bars 60 and enable the reposition unit 62 to perform parallel motion with respect to the supporting units 1. The plates 2 of the supporting units 1 are additionally interconnected through a rod 67 similar to the additional rod 42 shown in FIG. 9. The present device may also incorporate two or more reposition units 62.

The aforedescribed embodiment of the invention provides for a possibility of eliminating lateral vertical dislocation in spondylolisthesis.

The device, according to the invention, operates as follows.

In order to set the supporting unit 1 (FIG. 1) in position one should expose the vertebral arch and turn the screws 3 into the vertebral body 6 on both sides of the spinous process 4 as deep as the restrictor 8. Having denuded the spinous process 4 between the screws 3, one should pass two fixing pins 13 with the stops 24 through the spinous process 4 on both sides thereof in the planes substantially square with the vertebral axis and at an angle to the frontal plane so that the fixing pins 13 should cross over in the spinous process 4 with a gap therebetween aimed at reducing the local injury to the osseous tissue. Then the fork 12 together with one of its nuts 18 is fitted onto the spinous process 4 in such a manner that the prongs 15 should straddle the spinous process 4 and the fixing pins 13 should engage the slots 19. Whenever use is made of the fork 12 having the holes 22 in its prongs 15, first the fork 12 is to be set in position, then the fixing pins 13 are to be passed in a way described above.

The shank 14 of the fork 12 is guided to pass into the canal 16 of the plate 2 and is held with the other nut 18. The screws 3 are locked on the plate 2 with the aid of the brackets 11 fitted in the corresponding holes 5 and the nuts 10. The fixing pins 13 are tensioned properly and held to the plate 2 by means of the bolts 23 fitted in the corresponding holes 5, while the stops 24 rest upon the outer surfaces of the prongs 15 of the fork 12. Thus, the supporting unit is reliably connected to the vertebra in its anterior and posterior portions.

Whenever it becomes necessary to set the supporting unit 1 on two adjacent vertebrae, said unit may comprise two pairs of the screws 3 (FIG. 6) located on the opposite sides of the plates 2, as well as two means for grasping the spinous process of the vertebra (said means being omitted in the Drawing).

Having set the other supporting unit 1 in a way described above one should interconnect their plates 2 through two or more rods 25 (four such rods being represented in FIG. 6), aimed at adjusting the mutual position of the supporting units 1 and the nuts 26.

Whenever the device is applied in cases of spinal fractures without dislocation, the supporting units 1 are set above and below the fracture, the position of the spinal column is corrected and the spine is fixed in the corrected position by rigidly holding the plates 2 to the rods 25. When it becomes necessary to repair the affected osseous tissue by growing a bone regenerate of the vertebral body and arch so as to restore its shape both lengthwise and breadthwise, one should apply graduated distraction of the spine by displacing the supporting unit 1 towards the ends of the rods 25, using the nuts 26.

It is due to the use of such a device that an affected vertebra has been elongated by growing a bone regenerate without applying a bone graft for the first time in the world orthopedic practice.

Once the required curative effect has been attained and stabilized, the device is removed in the following order: first the nuts 26 are backed off and the rods 25 are removed, thereby disconnecting the supporting units 1. Then the screws 3 in each of the supporting units 1 are released and taken out. Whenever use is made of the forks 12 whose prongs 15 are provided with the slots 19, the ends of the fixing pins 13 held to the plate 2 are released, the plate 2 together with the fork 12 is removed, and the fixing pins 13 are pulled out. When the holes 22 are provided in the prongs 15 of the fork 12, the fixing pins 13 and the plate 2 with the fork 12 are removed in the reversed sequence. Next some single-stitch sutures are applied to the soft tissues remaining after removal of the device.

The device, according to the invention, as shown in FIG. 7 is adapted for elimination of lateral spinal curvatures. The supporting units 1 are to be set at the ends of the scoliotic arc of the spine in a way described above so that the crossarms 27 installed on the plates 2 should face each other. Then one should adjust the distance between the plates 2 and the crossarms 27 with the aid of the rods 28 and the nuts 29 so as to bring the holes in the brackets 30 in register, whereupon they are interconnected through a bolt so that the fulcrum of the thus-established hinge joint should pass through the apex of the scoliotic curvature. Then the plates 2 set angularly to each other are interconnected through the rods 25 by the nuts 26, and their mutual out-of-squareness is compensated for by means of the hinge joints 32. It is due to displacement of the nuts 26 towards the ends of one of the rods 25 located on the convex side of the scoliotic arc of the spine (the left-hand one in FIG. 7) that graduated distraction is applied to the concave side of the scoliotic arc. At the same time graduated compression is applied to the convex side of the scoliotic arc by displacing the nuts 26 towards the midpoint of the other rod 25 located on the convex side of the scoliotic arc.

Once the spinal column has assumed its normal position it is fixed in the corrected position with the aid of the device in order to stabilize the result thus attained, whereupon the device is removed and some sutures are applied to the soft tissues.

To attain a greater effect the flexible tie-member 37 is installed on the device of FIG. 7, using the shackle 34 held to the rod 25, which is located on the concave side of the scoliotic arc. The free end of the flexible tie-member 37 is fixed, by any heretofore-known technique, on the vertebra that is located at the apex of the scoliotic curvature. As gradiated distraction and compression are developed and applied to the respective spinal curvature sides with the aid of the rods 25, the apex of the spinal curvature is pulled towards the centre of curvature by virtue of graduated tensioning of the fle xible tie-member 37, using the rod 36 and the nuts 35.

For treatment of spinal fractures accompanied by dislocation of its portions in the frontal plane there is adapted the device, according to the invention, as shown in FIG. 8. To this end, the supporting units 1 are set above and below the fracture in such a manner that the crossarm 27 and the guideway 38 carrying the brackets 40 should face each other. The brackets 40 are held in the holes of the crossarm 27 by appropriately adjusting the amount of overhang of the rods 28 and 39 from the plates 2. Then the plates 2 are interconnected by the rods 25 and through the hinge joints 32 as it has been described above. By moving the brackets 40 carried by the crossarm 27, along the guideway 38 with the aid of the nuts 41, one can thereby effect the mutual parallel travelling of the supporting units 1, which in turn return the dislocated spinal portions to their normal position. When the spinal portions are dislocated lengthwise so that one of these overlaps the other, prior to holding the brackets 40 to the crossarm 27 one should return the overlapped vertebrae to their normal position by displacing the plates 2 towards the end of the rods 25, using the nuts 26. When it is necessary to increase the distraction force applied, or to add to the rigidity of the device, the additional rod 42 is set to the shank 14 of the forks 12 with the aid of the brackets 44 and the nuts 43, the effect of the rod 42 being similar to that of the rod 25. Once the spine has assumed its normal position the device is to be fixed in place until the end of the treatment period, whereupon it is removed.

The device, according to the embodiment of the invention as shown in FIG. 10 operates similarly to those described above with reference to FIGS 7 and 8 with the sole exception that it enables one to eliminate spinal curvatures both in the frontal and sagittal planes, as well as dislocations of the spinal portions at an angle to each other in cases of fractures, this being due to a possibility of angular displacements of the supporting units 1 both in the frontal and sagittal planes by virtue of the two-axis hinge joints 46 and 47. When it is necessary to exert a direct effect on the apex of the spinal curvature in the sagittal plane (that is, on the hunch) the additional supporting unit 1 is substituted for the crossarm 27.

The device illustrated in FIG. 11 is applicable for treatment of vertebral dislocation in the anteroposterior direction (spondylolisthesis). The supporting units 1 are set in this case in a way described hereinbefore, i.e., above and below the dislocated vertebra, while the reposition unit 51 is set on the disclocated vertebra itself. Then the supporting units 1 and the reposition unit 51 are interconnected through the respective rods 52, 53, 54 with the aid of which graduated spinal distraction is carried out, which is necessary for removal of the compensating forces applied to the dislocated vertebra by the nondislocated ones. Next the respective bar 48 is installed on the supporting units 1 and on the reposition unit 51. To relocate the dislocated vertebra back to its normal position, the reposition unit 51 receives graduated motion, with the aid of the nuts 50 and the shank 14a, in a required direction, while the rods 54 are moved simultaneously in the brackets 56. Once the spine has gained its normal position it is fixed in that position, with the aid of the device, in order to stabilize the result of treatment thus obtained, whereupon the device is removed.

In order to eliminate lateral spondylolisthesis use is made of the device illustrated in FIG. 12. The supporting units 1 are set on the spine on both sides of the disclocated vertebra, while the reposition unit 62 is located on the dislocated vertebra itself. Then the like ends of the units 1 and 62 are interconnected through the bars 60 at the ends of which the threaded rods 61 are provided, adapted to engage the holes in the plate 2. Next the supporting units 1 are displaced with the aid of the nuts 26 and the strips 59 so as to effect spinal distraction aimed at relieving the displaced vertebra from the compression load. Thereupon the reposition unit 62 receives graduated motion in a required direction by virtue of the nuts 66, the rods 65, the shackles 64 and the bolts 63. After relocation of the dislocated vertebra back to its proper position the spine is fixed with the aid of the device so as to stabilize the curative result obtained, whereupon the device is removed.

Whenever it is necessary to eliminate spondylolisthesis of a number of vertebrae, the reposition unit 62 of its own is to be placed on each of the affected vertebrae, and the treatment procedure mentioned above is performed.

The present invention is by no means limited to the embodiments of its implementation and application discussed hereinabove and therefore incorporates other various changes and modifications falling within the spirit and scope of the claims that follows.

The device, according to the invention, enables one to effect highly efficacious internal fixation of the spine, eliminate its deformities and restitute the proper shape of a vertebra by growing a bone tissue  generate), all this being due to the provision   reliable connection of the device to the vertei.  and due to a possibility of applying strictly graduated forces in any direction and at any stage of treatment.

## Industrial Applicability

The device proposed herein is applicable for restoration of the true shape of both an individual vertebra and the spinal column as a whole by resorting to the growth of the vertebral osseous tissue rather than to bone transplants.

## Claims

1. A device for treatment of spinal cu  ture and affections, having at least two supp  ng units (1) placed above the patient's skin, each of the supporting units comprising a plate (2), on which at least two screws (3) are movably mounted, the screws being adapted to be turned into the vertebral body, while the supporting units (1) themselves are interconnected through rods (25) aimed at adjusting the mutual position of the supporting units, CHARACTERIZED in that each of the plates (2) is provided with at least one means for grasping a spinous vertebral process (4), said means incorporating a two-prong fork (12) having a shank (14) interposed between the screws on the supporting plate with a possibility of a relative transverse motion in order to straddle the spinous process (4) by the fork prongs, and two fixing pins (13) passed through the spinous vertebral process (4) and through both prongs (15) of the fork (12), one of the ends of each pin being held in position on the plate (2), while the other end, on the prong at its extension from the fork.

2. A device as claimed in Claim 1, CHARACTERIZED in that a slot (19) is provided in either of the prongs (15) of the fork (12), said slot passing between the inner and outer surfaces of the prong (15) and being open towards the prong end, while longitudinal axes (20) of the slots (19) are inclined towards a longitudinal plane (21) of the fork (12) and are parallel to each other.

3. A device as claimed in Claim 1, CHARACTERIZED in that a hole (22) is made in each of the prongs (15) of the fork (12), said hole passing in between the inner and outer surfaces of the prong (15), while the longitudinal axes of the holes (22) are inclined towards the longitudinal plane (21) of the fork (12) and are parallel to each other.

4. A device as claimed in any one of Claims 1 to 3, CHARACTERIZED in that a crossarm (27) is installed on each plate (2) of the supporting unit (1) parallel to said plate and is connected thereto by means of rods (28) for adjusting the distance between said plates, said crossarms (27) being situated on the opposite sides of the plates (2) and being interconnected with a possibility of mutual displacement, and the rods (28) for adjusting the mutual position of the plates (2) are connected thereto by means of hinge joints (32).

5. A device as claimed in Claim 4, CHARACTERIZED in that the crossarms (27) are interconnected with a possibility of mutual angular displacement in the frontal plane.

6. A device as claimed in Claim 4, CHARACTERIZED in that one of the crossarms (27) is shaped as a guideway (38), while the other crossarm is connected to the guideway with a possibility of mutual parallel motion.

7. A device as claimed in Claim 6, CHARACTERIZED in that the guideway (38) and the other crossarm are interconnected with a possibility of mutual angular motion in the frontal and sagittal planes.

8. A device as claimed in Claim 5 or 7, CHARACTERIZED in that it comprises a flexible tie-member (37) one of whose ends is rigidly coupled to the vertebra, while the other end is connected to the supporting units with a possibility of adjusting the tension of said flexible tie-member (37) in the direction of the vertex of an angle confined between the plates of the supporting units (1) and lying in the frontal plane.

9. A device as claimed in Claim 6 or 7, CHARACTERIZED in that it comprises an additional rod (42) for adjusting the mutual position of the plates (2), the ends of said rod being installed on the shanks (14) of the forks (12) with a possibility of mutual displacement.

10. A device as claimed in any one of Claims 1 to 3, CHARACTERIZED in that the plates (2) of the supporting units (1) are rigidly interconnected through a bar (48), while at least one reposition unit (51) is interposed between the supporting units (1), said reposition unit being substantially similar to the supporting unit (1) and being installed on the bar (48) with a possibility of performing anteroposterior motion, and the plate (2a) of the reposition unit (51) is connected to the supporting plates (2) of the supporting units (1) through rods (52, 53, 54) adapted for adjusting their mutual position.

11. A device as claimed in any one of Claims 1 to 3, CHARACTERIZED in that the like ends of the plates (2) of the supporting units (1) are interconnected through bars (60) at whose ends rods (61) are provided for adjusting the mutual position of the plates (2), while at least one reposition unit (62) is interposed between the supporting units (1), said reposition unit being similar to the supporting unit (1), and the ends of the plate (2a) of the reposition unit (62) are connected to the respective bars (60) with a possibility of parallel motion of the reposition unit (62) with respect to the supporting units (1).

FIG.1

14

15

19

**FIG.2**

20    20    19

21

**FIG.3**

14

15

22

**FIG.4**

14

15

22

**FIG.5**

FIG. 6

EP 0 418 387 A1

FIG.7

13

FIG. 8

FIG. 9

FIG.10

FIG.11

FIG.12

# INTERNATIONAL SEARCH REPORT

International Application No PCT/SU 89/00046

## I. CLASSIFICATION OF SUBJECT MATTER (if several classification symbols apply, indicate all) 6

According to International Patent Classification (IPC) or to both National Classification and IPC

Int.Cl.$^5$ – A61B 17/60

## II. FIELDS SEARCHED

### Minimum Documentation Searched 7

| Classification System | Classification Symbols |
|---|---|
| Int.Cl.$^5$ | A61B 17/56, 17/58, 17/60 |

### Documentation Searched other than Minimum Documentation to the Extent that such Documents are Included in the Fields Searched 8

## III. DOCUMENTS CONSIDERED TO BE RELEVANT 9

| Category * | Citation of Document, 11 with indication, where appropriate, of the relevant passages 12 | Relevant to Claim No. 13 |
|---|---|---|
| A | SU, A1, 1063405 (KOSTNO-TUBERKULEZNY SANATORY "URAL"), see the claims, figures 1,2 | 1-3 |
| A | SU, A1, 1237194 (KOSTNO-TUBERKULEZNY SANATORY "URAL" see the claims, figures 1,3,7 | 4-7,9-11 |
| A | SU, A1, 839515 (KOSTNO-TUBERKULEZNY SANATORY "URAL" see the claims, figures 1,2 | 8 |

* Special categories of cited documents: 10

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

## IV. CERTIFICATION

| Date of the Actual Completion of the International Search | Date of Mailing of this International Search Report |
|---|---|
| 28 September 1989 (28.09.89) | 30 October 1989 (30.10.89) |
| International Searching Authority<br><br>ISA/SU | Signature of Authorized Officer |